# EUROPÄISCHE PATENTSCHRIFT

(11) **EP 2 068 787 B1**
(45) Veröffentlichungstag und Bekanntmachung des Hinweises auf die Patenterteilung: **04.01.2012**
(21) Anmeldenummer: 07820785.9
(22) Anmeldetag: 01.10.2007
(51) Int. Cl.: A61F 5/08

(54) **VORRICHTUNG ZUM UMFORMEN VON KNOCHEN**
DEVICE FOR RESHAPING BONES
DISPOSITIF POUR DÉFORMER DES OS

(30) Priorität: 02.10.2006 EP 06121632
(43) Veröffentlichungstag der Anmeldung: 17.06.2009
(73) Patentinhaber: Honegger, Norina, 8704 Herrliberg (CH)
(72) Erfinder: Honegger, Norina, 8704 Herrliberg (CH)
(74) Vertreter: Dr. Graf & Partner AG
(86) Internationale Anmeldenummer: PCT/EP2007/060403
(87) Internationale Veröffentlichungsnummer: WO 2008/040708

(56) Entgegenhaltungen:
- DE-B- 1 104 655
- DE-C- 76 055
- DE-C- 321 737
- DE-C- 811 255
- US-A- 3 742 943

## Beschreibung

Die Erfindung betrifft eine Vorrichtung zum Umformen von Knochen beziehungsweise zur Beeinflussung des Knochenwachstums, insbesondere des Nasenbeins. Die Erfindung betrifft weiter ein Verfahren zum Herstellen einer Vorrichtung zum Umformen von Knochen.

### Stand der Technik

Die Form der Nase ist für ein attraktives Gesichtsprofil von zentraler Bedeutung. Idealerweise ist die Nase harmonisch verlaufend in den Gesichtszügen eingebettet. Weist die Nase jedoch eine eigenwillige Form und/oder eine überproportionale Grösse auf, so wird die Nase unweigerlich zum Blickfang und dadurch zum dominanten Teil des Gesichtes.

Die Form der Nase kann heute üblicherweise durch einen verhältnismäßig kleinen, aber dennoch sehr anspruchsvollen chirurgischen Eingriff dauerhaft korrigiert werden. In über 80 Prozent der Eingriffe wird die Nase verkleinert, in den übrigen die Nase vergrößert.

Im Wesentlichen besteht die Nase aus knöchernen und knorpeligen Anteilen, die ein pyramidenförmiges Gerüst unter der bedeckenden Nasenhaut bilden. Senkrecht in der Mitte befindet sich die Nasenscheidewand, die im vorderen Anteil knorpelig, im hinteren Anteil knöchern ist. Bei Nasenoperationen gilt es, dieses Gerüst so zu gestalten, dass sich die äußere Form der Nase durch die bedeckende Haut verändert. Die Rhinoplastik kann die Nase verkleinern oder vergrößern, die Form des Nasenrückens sowie des Naseneinganges mit den Nasenflügeln und den Nasenlöchern korrigieren, sowie die Länge der Nase und den Winkel zwischen Nase und Oberlippe verändern. Der Eingriff wird insbesondere aus ästhetischen Gründen zur Harmonisierung der Nase und des gesamten Gesichtes (ästhetische Rhinoplastik) durchgeführt. Damit der Eingriff keine sichtbaren Spuren hinterlässt, werden die Hautschnitte im Naseninneren oder am Nasensteg angelegt. Durch diesen Zugang modelliert der Arzt das Knochen- und Knorpelgerüst der Nase je nach Zielsetzung vorteilhaft um. So kann die Nase zum Beispiel verkleinert, verschmälert oder begradigt werden. Eine derartige Korrektur der Nase sollte erst nach Abschluss des Knochenwachstums, also etwa ab dem 16. oder 17. Lebensjahr vorgenommen werden.

Dieses bekannte Verfahren weist die Nachteile auf, dass ein anspruchsvoller chirurgischer Eingriff mit all den damit verbundenen Risiken erforderlich ist, dass der Eingriff sehr teuer ist und üblicherweise von den Krankenkassen nicht finanziert wird, und dass das Verfahren aus finanziellen Gründen nur einer begrenzten Bevölkerung zugänglich ist.

Die Druckschrift DE 321737 (Basis für den Oberbegriff des Anspruchs 1) offenbart einen Nasenformer, der aus einem Hüllkörper mit einstellbarem Druckteil besteht. Der Nasenformer wird am Kopf befestigt und das Druckteil soll einen Druck auf die Nase ausüben. Dieser Nasenformer weist die Nachteile auf, dass die genaue Lage des Druckteils nicht genau bestimmbar ist, dass sich die Lage des Druckteils während dem Tragen des Nasenformers verändern kann, und dass der Nasenformer äusserst unangenehm zu tragen ist, und daher für ein längeres und wiederholtes Tragen, insbesondere auch während des Schlafens, nicht geeignet ist.

Die Druckschrift DE 811 255 offenbart ein brillenähnliches Gerät zum Nasenformen. Dieses Gerät weist den Nachteil auf, dass sich die Lage des Druckteils während des Tragens verändern kann, dass die maximal vom Druckteil erzeugbare Druckkraft zu gering ist, und dass der Nasenformer äusserst unangenehm zu tragen ist, und daher für ein längeres und wiederholtes Tragen, insbesondere auch während dem Schlafen, nicht geeignet ist.

Alle bekannten Nasenformer weisen den Nachteil auf, dass sich deren Lage während des Tragens verändern kann, das heisst, dass sie bezüglich der Nase verrutschen. Zudem ist das Tragen unangenehm und lästig.

### Darstellung der Erfindung

Aufgabe der Erfindung ist es, eine Vorrichtung, sowie ein Verfahren zum Herstellen der Vorrichtung vorzuschlagen, welche ein kostengünstiges und sicheres Umformen von Knochen, und insbesondere eine Korrektur der Nasenform ermöglicht.

Diese Aufgabe wird gelöst mit einer Vorrichtung zum Umformen von Knochen oder Knorpel aufweisend die Merkmale von Anspruch 1. Die Unteransprüche 2 bis 9 betreffen weitere, vorteilhafte Ausgestaltungen. Die Aufgabe wird weiter gelöst mit einem Verfahren zum Herstellen der erfindungsgemässen Vorrichtung aufweisend die Merkmale von Anspruch 10. Die Aufgabe wird weiter gelöst mit einem Bausatz aufweisend die Merkmale der Ansprüche 11 bis 15.

Die Aufgabe wird insbesondere gelöst mit einer Vorrichtung zum Umformen von Knochen beziehungsweise zum Beeinflussen des Wachstums von Knochen oder Knorpel, insbesondere Nasenknochen, umfassend eine Formvorrichtung mit einer zur Auflage auf die Haut bestimmten Anpressfläche, und umfassend eine Haltevorrichtung, welche mit der Formvorrichtung verbindbar ist, wobei die Haltevorrichtung sowie die Formvorrichtung derart zusammenwirkend ausgestaltet ist, dass die Formvorrichtung zumindest an der Anpressfläche unter Ausübung einer Anpresskraft an der Haut anliegen kann.

Die Aufgabe wird weiter insbesondere gelöst mit einer Vorrichtung zum Umformen von Knochen, insbesondere Nasenknochen, umfassend eine Formvorrichtung mit einem Anpressteil, das eine zur Auflage auf die Haut bestimmten Anpressfläche aufweist, und eine Haltevorrichtung, welche mit der Formvorrichtung verbindbar ist, wobei die Haltevorrichtung sowie die Formvorrichtung derart zusammenwirkend ausgestaltet ist, dass die Formvorrichtung zumindest an der Anpressfläche unter Ausübung einer Anpresskraft an der Haut anliegen kann, wobei die Formvorrichtung einen Träger mit einer Auflagefläche umfasst, und wobei der Träger derart verlaufend ausgestaltet ist, dass die Auflagefläche zumindest entlang eines Teilabschnittes der Aussenkontur der Nasenwurzel folgt und über die Auflagefläche am Gesicht aufliegen kann.

Unter Knochen werden in der vorliegenden Schrift auch knochenähnliche Strukturen oder Knorpel verstanden.

Es hat sich herausgestellt, dass der Knochen des Menschen während des gesamten Lebens einem ständigen Umbauprozess unterworfen ist. Beim normalen Umbau des Knochens besteht ein Gleichgewicht zwischen osteoklastischer und osteoblastischer Aktivität. Eine dauernde Belastung des Knochens führt jedoch zu Anpassungsvorgängen wie Corticalisverdickung und Ausrichtung der Spongiosabälkchen. Wird die individuelle Belastungstoleranz durch wiederholte submaximale gleichartige Reize überschritten, finden im Bereich mechanischer Spannungsspitzen Strukturveränderungen und Umbauvorgänge statt. Die vorliegende Erfindung macht sich diesen Effekt zu nutzen, indem auf gewisse Knochenbereiche ein derartiger Druck erzeugt wird, sodass in diesem Knochen eine Spannung entsteht, welche die Ausformung des Knochens oder Knorpels beeinflusst. Dazu wird auf die Haut, welche sich über dem genannten Knochenbereich befindet, eine Kraft ausgeübt, welche sich über die Haut auf den Knochen fortpflanzt, sodass mit der erfindungsgemässen Formvorrichtung die Form des Knochens beeinflusst wird. Die erfindungsgemässe Vorrichtung wird vorzugsweise bei sich im Wachstum befindlichen Kindern verwendet, da die Ausgestaltung des Knochens, insbesondere des Nasenknochens, in dieser Phase relativ einfach beeinflussbar ist. Die erfindungsgemässe Vorrichtung ist jedoch auch für erwachsene Menschen verwendbar. Die erfindungsgemässe Vorrichtung wird vorzugsweise während mehreren Stunden pro Tag angewendet, beispielsweise während des Schlafs.

Um beispielsweise die Nasenform zu korrigieren oder umzuformen wird die erfindungsgemässe Vorrichtung vor dem Schlafen gehen montiert, indem die Formvorrichtung auf die Nase gelegt wird, und die Formvorrichtung mit einer Haltevorrichtung am Kopf fixiert wird. Die Formvorrichtung weist Anpressflächen auf, welche derart ausgerichtet, angeordnet und ausgestaltet sind, dass während dem Schlafen auf eine vorherbestimmte Stelle des Nasenknochens eine Presskraft ausgeübt wird.

Durch üblicherweise mehrmonatiges bis jahrelanges Tragen der erfindungsgemässen Vorrichtung während des Schlafens verändert sich die Form des Nasenknochens, beziehungsweise der wachsende Nasenknochen gleicht sich einer vorgegebenen Form an. Dies wird vorzugsweise während der Wachstumsphase angewendet, damit der Nasenknochen nach Abschluss des Knochenwachstums, das heisst nach dem 16. bis 17. Lebensjahr, die gewünscht Nasenknochenform aufweist und diese auch beibehält.

In einer vorteilhaften Ausgestaltung weist die Vorrichtung einen Träger auf, welcher eine Auflagefläche ausbildet. Die Auflagefläche verläuft vorzugsweise zumindest über einen Teilabschnitt entlang der Nasenwurzel und liegt dabei, vorzugsweise mit nur geringer oder vernachlässigbar kleiner Anpresskraft am Gesicht auf. Die derart ausgestaltete Auflagefläche hat zur Folge, dass die Vorrichtung eine relativ genau definierte Lage aufweist und bezüglich der Nase kaum verrutscht. Dies ist besondere bei langfristigem Tragen und beim Tragen während dem Schlafen von besonderer Bedeutung.

In einer besonders vorteilhaften Ausgestaltung ist der Träger über eine erste Haltevorrichtung am Kopf fixierbar, wobei das Anpressteil über eine zusätzliche zweite Haltevorrichtung am Kopf fixierbar ist.

Diese Ausgestaltung weist den Vorteil auf, dass die Lage der Vorrichtung bezüglich der Nase im Wesentlichen durch den Träger und die Haltevorrichtung bestimmt ist, wogegen der genaue Ort der Krafteinleitung durch die Lage des Anpressteils und die Grösse der eingeleiteten Kraft durch die zweite Haltevorrichtung bestimmt ist. Diese Ausgestaltung weist den Vorteil auf, dass der Tragkomfort der Vorrichtung als angenehm empfunden wird, dass kaum ein Verrutschen zwischen Vorrichtung und Nase auftritt, sodass der Ort der Krafteinleitung in die Nase reproduzierbar und langfristig konstant ist, und dass die Grösse der auf die Nase ausgeübten Kraft über die zweite Haltevorrichtung einstellbar ist. Die Einstellbarkeit der ausgeübten beziehungsweise auf die Nase bewirkten Kraft ist für einen langfristigen Tragkomfort von entscheidender Bedeutung, denn das subjektive Empfinden individueller Menschen ist sehr unterschiedlich, und kann sich selbst bei einem Individuum verändern. Die Einstellbarkeit der auf die Nase wirkenden Kraft weist daher den Vorteil auf, dass die Vorrichtung immer derart am Kopf fixiert oder nachgestellt werden kann, dass ein unangenehmer Tragkomfort vermieden oder reduziert werden kann. Dadurch kann insbesondere verhindert werden, dass die Nase unangenehm zu schmerzen beginnt. Dadurch kann zudem ein langfristiges Tragen der Vorrichtung gewährleistet werden.

In einer besonders vorteilhaften Ausgestaltung ist die Vorrichtung einstückig ausgestaltet, insbesondere als leichtes Kunststoffteil.

In einer weiteren besonders vorteilhaften Ausgestaltung ist die Vorrichtung als Bausatz ausgestaltet, umfassend eine Mehrzahl von pflasterähnlichen Auflagen, welche in die Formvorrichtung geklebt werden können, um dadurch die Anpressfläche auszubilden. Der Bausatz umfasst eine Mehrzahl von Auflagen, welche sich insbesondere bezüglich Dicke und/oder Fläche unterscheiden, sodass je nach der angestrebten Anpressfläche eine entsprechende Auflage ausgewählt und in die Formvorrichtung geklebt werden kann. Die erfindungsgemässe Vorrichtung wird nachfolgend am Beispiel der Umformung der Nase im Detail erläutert. Die erfindungsgemässe Vorrichtung ist jedoch zur Umformung beinahe jedes Knochens des Menschen geeignet, beispielsweise auch zur Umformung des Kinns oder der Backenknochen. Die Erfindung wird nachfolgend an Hand von Ausführungsbeispielen beschreiben.

### Kurze Beschreibung der Zeichnungen

Die zur Erläuterung der Ausführungsbeispiele verwendeten Zeichnungen zeigen:
- Fig. 1: eine montierte Vorrichtung zum Umformen von Knochen;
- Fig. 2: die in Figur 1 dargestellte Vorrichtung im Detail;
- Fig. 3: einen Längsschnitt durch die in Figur 2 im Schnitt dargestellte, einstückige Formvorrichtung;
- Fig. 4: ein weiteres Ausführungsbeispiel einer Formvorrichtung;
- Fig. 5: einen Längsschnitt durch die in Figur 4 dargestellte und auf eine Nase aufgesetzte Formvorrichtung;
- Fig. 15: ein weiteres Ausführungsbeispiel eines Anpressteils;
- Fig. 16: ein weiteres Ausführungsbeispiel einer Formvorrichtung;
- Fig. 17: einen Querschnitt durch ein weiteres Ausführungsbeispiel eines Anpressteils;
- Fig. 18: eine Vorrichtung zum Herstellen der Vorrichtung zum Umformen von Knochen;
- Fig. 19: ein weiteres Ausführungsbeispiel einer einstückigen Formvorrichtung;
- Fig. 20: ein Schnitt durch die in Figur 19 dargestellte Formvorrichtung entlang der Schnittlinie A-A;
- Fig. 21: eine Seitenansicht eines weiteren Ausführungsbeispieles einer Formvorrichtung;
- Fig. 22: ein Längsschnitt durch die in Figur 21 dargestellte Formvorrichtung;
- Fig. 23: eine Draufsicht auf ein Anpressteil von Innen;
- Fig. 24a - 24d: unterschiedlich ausgestaltete Auflagen;
- Figur 25a - 25c: Querschnitte durch unterschiedliche ausgestaltete Auflagen;
- Fig. 26: einen Querschnitt durch eine Formvorrichtung;
- Fig. 27: ein weiteres Ausführungsbeispiel einer einstückigen Formvorrichtung;
- Fig. 28: eine Seitenansicht eines weiteren Ausführungsbeispieles einer Formvorrichtung;
- Fig. 29: einen Querschnitt durch eine Formvorrichtung.

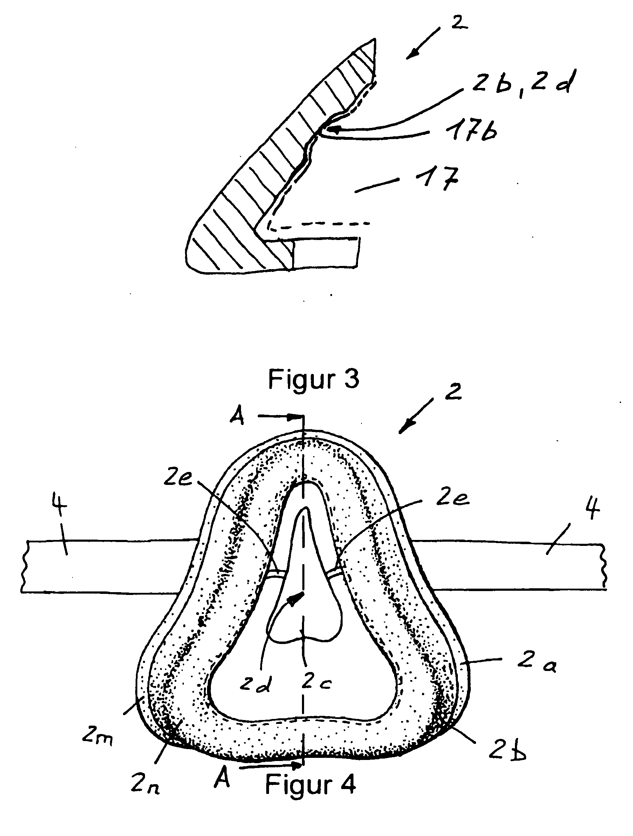

Grundsätzlich sind in den Zeichnungen gleiche Teile mit gleichen Bezugszeichen versehen. Fig. 4-5 und 15-29 offenbaren Ausführungsformen der Erfindung.

### Wege zur Ausführung der Erfindung

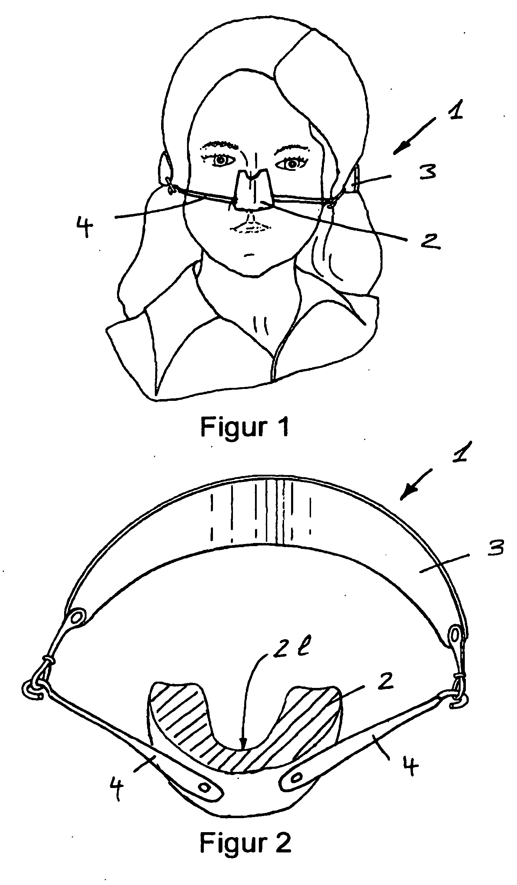

Fig. 1 zeigt eine am Kopf eines Teenagers fixierte Vorrichtung 1 zum Umformen von Nasenknochen beziehungsweise zur Beeinflussung der Form der Nase. Die Vorrichtung umfasst eine Formvorrichtung 2, welche über ein Verbindungsmittel 4 mit einer hinten um den Kopf verlaufenden Haltevorrichtung 3 verbunden ist. Diese Vorrichtung 1 wird üblicherweise über Nacht am Kopf fixiert, sodass die Formvorrichtung 2 während beispielsweise etwa acht Stunden eine Anpresskraft auf die Haut und den darunter liegenden Nasenknochen ausübt.

Figur 2 zeigt die in Figur 1 dargestellte Vorrichtung 1 im Detail. Die Formvorrichtung 2 ist in einem Querschnitt dargestellt, sodass die zur Aufnahme der Nase bestimmte Ausnehmung 21 ersichtlich ist. Die Formvorrichtung 2 ist einstückig ausgestaltet und besteht somit aus einem einzigen Teil. Die Haltevorrichtung 3 ist vorzugsweise elastische und die Verbindungsmittel 4 sind vorzugsweise starr ausgestaltet, damit die Formvorrichtung 2 mit genügend grosser Kraft gegen die Nase gepresst wird und verrutschfest bleibt, insbesondere während dem Schlaf. Die Haltevorrichtung 3 kann auf unterschiedlichste Weise ausgestaltet sein, um am Kopf befestigt zu werden. Beispielsweise kann die Haltevorrichtung 3 auch zwei oder drei hinter dem Kopf verlaufende Bänder aufweisen, um einen besseren Halt zu bewirken.

Figur 3 zeigt einen Längsschnitt durch die in Figur 2 dargestellte Formvorrichtung 2. Nur schematisch und gestrichelt ist der Verlauf der Nase 17 dargestellt. Die Nase weist einen Höcker 17b auf. Die Formvorrichtung 2 weist zumindest eine zur Auflage auf die Haut bestimmte Auflagefläche 2b mit Anpressfläche 2d auf, sodass die von der Formvorrichtung 2 ausgeübte Anpresskraft vorzugsweise zum grossen Teil über die Anpressfläche 2d erfolgt. Die Auflagefläche 2b ist daher derart bezüglich Ausrichtung und/oder Grösse und/oder Oberflächenverlauf ausgestaltet, dass die über die Anpressfläche 2d erzeugte Kraft auf eine bestimmte Hautstelle und somit auf einen vorherbestimmten Knochenbereich 17b einwirkt, um dessen Form und/oder Wachstum zu beeinflussen. Die Anpressfläche 2d kann auf unterschiedlichste Weise angeordnet und ausgestaltet sein, und beispielsweise auch aus zwei oder mehreren Teilflächen bestehen, oder beispielsweise entlang der gesamten Länge des Nasensattels oder entlang der Seitenkonturen verlaufen. Die Anordnung, Anzahl, Grösse und Ausrichtung der Anpressflächen 2d ist bestimmt durch die Lage der Knochen- beziehungsweise Knorpelbereiche, deren Form und/oder Wachstum beeinflusst werden sollten. Daher ist es auch wichtig, dass die Formvorrichtung 2 während vorzugsweise einer grösseren Anzahl Anwendungen reproduzierbar gleich oder ähnlich angeordnet auf die Nase aufgesetzt werden kann, damit die Anpresskraft auf dieselben Knochenbereiche ausgeübt wird. Daher kann es sich als vorteilhaft erweisen, die Formvorrichtung 2 mit einer derartig ausgestalteten Ausnehmung 21 zu versehen, dass die Formvorrichtung 2 quasi selbst zentrierend auf die Nase aufgesetzt werden kann. Die Formvorrichtung 2 ist beispielsweise aus Kunststoff gefertigt, wobei die Formvorrichtung 2 auch lokal unterschiedliche Elastizitäten aufweisen kann.

Die Figuren 1 bis 3 dienen zum Verständnis der Erfindung, wobei die in den Figuren 1 bis 3 offenbarte Ausgestaltung der Formvorrichtung 2 nicht Teil der vorliegenden Erfindung ist.

In einer vorteilhaften Ausgestaltung umfasst die Formvorrichtung 2, wie in Figur 4 dargestellt, einen Träger 2a mit anatomisch ausgestalteter Auflagefläche 2b, wobei der Träger 2a mit den Verbindungsmitteln 4 verbunden ist. Der Träger 2a besteht beispielsweise aus einem metallischen Rahmen 2m sowie einem elastischen Kunststoff 2n, welcher die Auflagefläche 2b ausbildet. Im dargestellten Ausführungsbeispiel entspricht der Verlauf der Innenkontur des Trägers 2a dem Verlauf der Aussenkontur der Nasenwurzel, sodass der Träger 2a derart am Gesicht angelegt werden kann, dass der Träger 2a die Nasen umschliesst und im Bereich der Nasenwurzel über die Auflagefläche 2b am Gesicht anliegt. Dieses grossflächige und durch die Lage der Nase bestimmte

Anlegen des Trägers 2a weist den Vorteil auf, dass der Träger 2a reproduzierbar gleich und verrutschfest oder im Wesentlichen verrutschfest, ähnliche wie in Figur 1 dargestellt, angelegt werden kann. Der Träger 2a beziehungsweise dessen Metallrahmen 2m bildet somit eine Basisteil, an welchem ein oder mehrere Anpressteile 2c befestigt werden können. Im dargestellten Ausführungsbeispiel ist ein Anpressteil 2c über zwei als Stege ausgebildete Haltemittel 2e fest mit dem Träger 2a verbunden. Das Anpressteil 2c weist eine Anpressfläche 2d auf. Es könnten auch mehrere Anpressteile 2c mit dem Träger 2a verbunden sein. Das Anpressteil 2c könnte auch nur mit einem einzigen Haltemittel 2e mit dem Träger 2a verbunden sein. Die Anpressteile 2c könnten bezüglich dem Haltemittel 2e auch verschiebbar und/ oder lösbar und wieder fixierbar angeordnet sein. Der in Figur 4 dargestellte Träger 2a kann somit auf unterschiedlichste Weise mit Anpressteilen 2c bestückt werden.

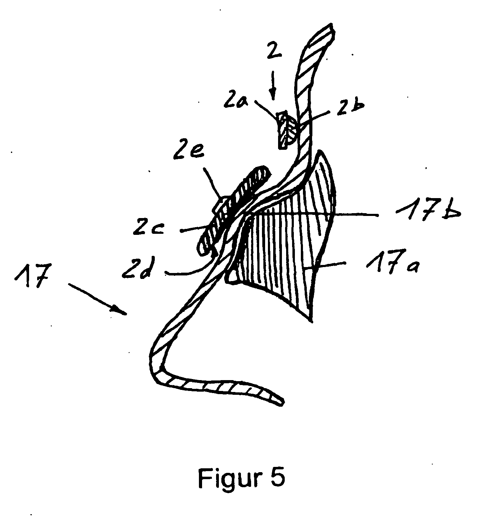

Figur 5 zeigt schematisch einen Längsschnitt durch eine Nase 17 mit Nasenknochen 17a. Auf die Nase 17 ist die in Figur 4 dargestellte Formvorrichtung 2 aufgesetzt. Das Anpressteil 2c mit Anpressfläche 2d liegt am Nasenhöcker 17b an, und übt eine auf den Nasenhöcker 17b beziehungsweise auf den Nasenknochen 17a wirkende Anpresskraft aus. Das Anpressteil 2c beziehungsweise die Anpressfläche 2d kann, wie bereits mit Figur 3 beschrieben, abhängig von der jeweiligen anatomischen Form der Nase, in einer Vielzahl von Möglichkeiten ausgestaltet und angeordnet sein. Es können auch mehrere Anpressflächen 2d oder auch eine Mehrzahl von Anpressteilen 2c vorgesehen sein.

Figur 2 zeigt ein Ausführungsbeispiel eines Trägers 2a mit einem runden, metallischen Rahmen oder einem Kunststoffrahmen 2m sowie einem den Rahmen 2m umschliessenden elastischen Kunststoff 2n. Der metallische Rahmen oder der Kunststoffrahmen 2m kann in einer vorteilhaften Ausführungsform als biegsam ausgestaltet sein, sodass der Verlauf des Rahmens 2m beispielsweise dem anatomischen Verlauf der Nase beziehungsweise der Nasenwurzel entsprechend angepasst werden kann. Jeder Schenkel des Trägers 2a kann auch über je einen sogenannten Gesichtsbogen 5 mit einer Haltevorrichtung 3 verbunden. Die beiden Gesichtsbögen 5 können an deren Kreuzungsstelle fest miteinander verbunden.

In einer vorteilhaften Ausgestaltung steht beim Anpassen der Formvorrichtung 2 ein Bausatz beziehungsweise ein Kit zur Verfügung, welcher eine Mehrzahl von Trägern 2a unterschiedlicher Grösse und/oder Gestalt, sowie eine Mehrzahl von Anpressteilen 2c, vorzugsweise auch unterschiedlicher Grösse und/oder Gestalt, sowie eine Mehrzahl unterschiedlicher Gesichtsbogen 5 umfasst. Ein derartiger Bausatz ermöglicht es eine Vielzahl unterschiedlich ausgestalteter, individuell an die Anatomie eines Menschen angepasste Formvorrichtungen 2 zu bilden.

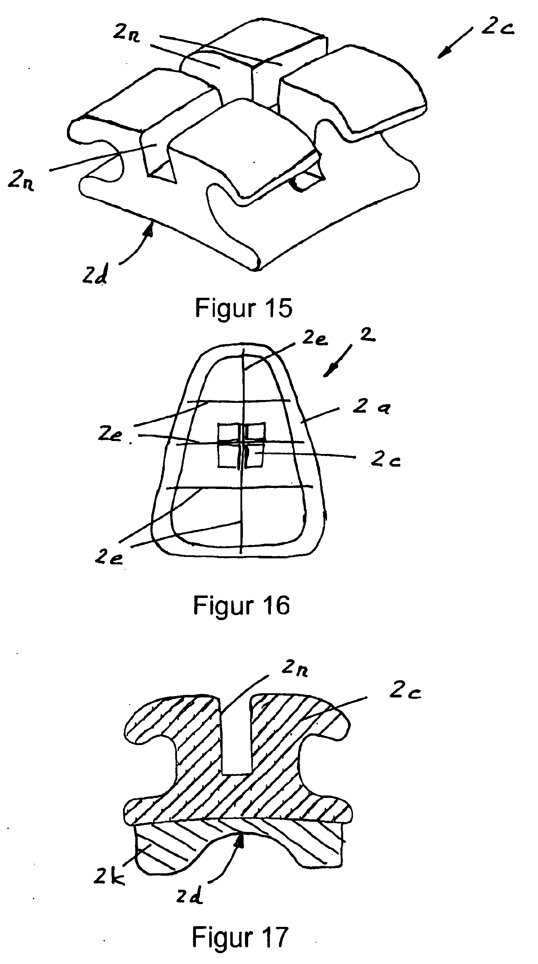

Am Träger 2a könnten auch, wie in Figur 16 in einer Draufsicht dargestellt, eine Mehrzahl von Haltemitteln 2e angeordnet sein, welche lösbar oder fest mit dem Träger 2a verbunden sind. Die Haltemittel 2e können auch gitterförmig ausgestaltet sein, mit zum Beispiel fest verbundenen Kreuzungspunkten. Die Haltemittel 2e sind vorteilhafterweise als dünner Draht von beispielsweise 1 mm, oder 0.5 mm bis 1.5 mm, Durchmesser ausgestaltet, wobei das Gitter derart gewölbt ist, dass dieses bei aufgesetzter Formvorrichtung 2 bezüglich der Nase beabstandet verläuft. Ein derartiger Träger 2a mit Haltemittel 2e kann ein sehr geringes Gewicht aufweisen. Vorteilhafterweise wird, wie in Figur 15 dargestellt, ein Anpressteil 2c verwendet, der sich kreuzende Nuten 2n aufweist. Dieses Anpressteil 2c wird, wie in Figur 16 dargestellt, vorteilhafterweise an sich kreuzenden Haltemitteln 2e mit diesen verbunden, z.B. durch Löten oder Kleben, sodass das Anpressteil 2c in einer genau definierten Lage im Gitter beziehungsweise in der Formvorrichtung 2 gehalten ist. Falls erforderlich kann das Anpressteil 2c auch durch ein anderes Anpressteil 2c ersetzt werden. Es wäre jedoch auch ein Anpressteil 2c ohne kreuzende Nuten geeignet, welcher am Gitter befestigt werden kann. Es könnten auch eine Mehrzahl von Anpressteilen 2c am Gitter befestigt werden.

Figur 17 zeigt einen Querschnitt durch ein weiteres Ausführungsbeispiel eines Anpressteils 2c. Im Unterschied zu dem in Figur 15 dargestellten Ausführungsbeispiel weist das Anpressteil 2c gemäss Figur 17 zusätzlich ein Formteil 2k auf, welche die Anpressfläche 2d ausbildet.

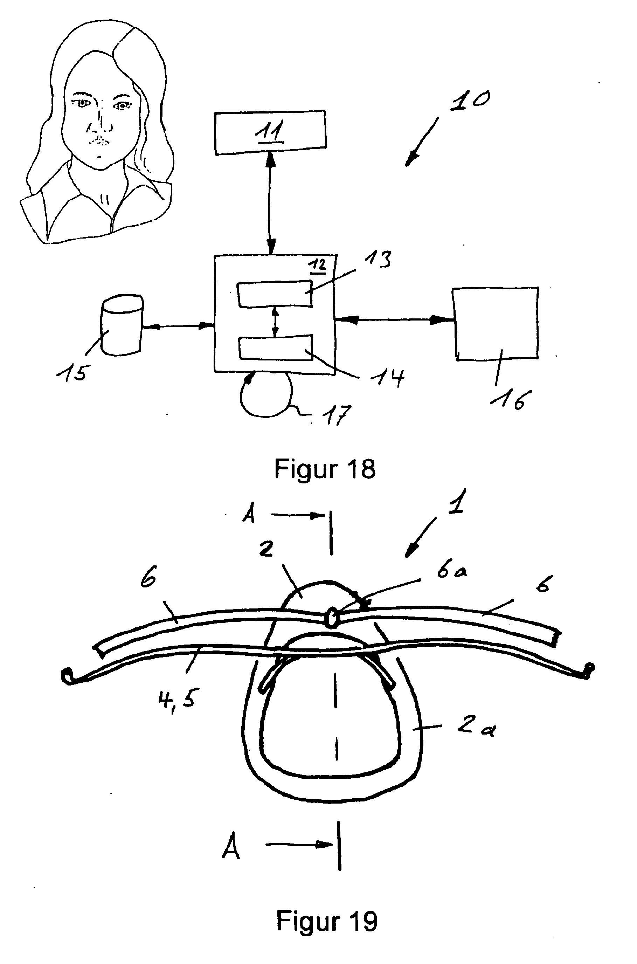

Figur 18 zeigt schematisch eine Vorrichtung 10 zum Herstellen der Formvorrichtung 2. Im dargestellten Ausführungsbeispiel wird die Erstellung einer Formvorrichtung 2 zum Umformen der Nase dargestellt. Dieselbe Vorrichtung 10 ist natürlich auch zum Erstellen von Formvorrichtungen 2 für andere Körperteile geeignet. Die Form der Nase wird mit einem 3-dimensionalen Scanner 11 abgetastet und die Daten einem Server 12 übermittelt. Im Speicher 14 wird ein 3-dimensionales Datenmodell der ausgemessenen Form der Nase bespeichert. Daraufhin kann mit Hilfe eines interaktiven Verfahrens 17 ein 3-dimensionales Datenmodell der gewünschten Nasenform gespeichert werden. Der Server hat Zugriff auf eine Datenbank 15, in welcher die Daten einer Mehrzahl von Formvorrichtungen 2, Trägern 2a, Anpressteile 2c usw. gespeichert sind. Das im Server 12 gespeicherte Verarbeitungsmodul 13 ist in der Lage, basierend auf den genannten, zur Verfügung stehenden Daten den Aufbau einer geeigneten Formvorrichtung 2 vorzuschlagen. Die gesamte Formvorrichtung 2 kann beispielsweise basierend auf Standartkomponenten zusammengestellt werden. In einer bevorzugten Ausgestaltung wird der Oberflächenverlauf des Formteils 2k jedes Anpressteils 2c individuell ausgestaltet, indem beispielsweise der Server 12 mit einer Maschine 16 verbunden ist, welche ein Formteil 2k mit individuell verlaufender Oberfläche 21 herzustellen erlaubt. In einer vorteilhaften Ausgestaltung sind die Anpressteile 2c in ihrer Grundform mit einem konstant dicken Formteil 2k versehen. Dieses Formteil 2k kann in der Maschine 16 individuell bearbeitet werden, sodass das derart gefertigte Formteil 2k eine individuell verlaufende Oberfläche 21 aufweist, mit zumindest einer Anpressfläche 2d. Das zumindest eine Anpressteil 2c wird daraufhin am Träger 2a befestigt, sodass die Formvorrichtung 2 ausgebildet wird, welche daraufhin mit einer Vorspannkraft an die Nase angelegt werden kann, um deren Form zu beeinflussen.

Das Verfahren zum Herstellen einer Formvorrichtung umfasst in einer bevorzugten Ausgestaltung die folgenden Schritte:
a) Berührungsloses Abtasten der zu verändernden Hautoberfläche und Erstellen eines digitalen Modells des Verlaufs der Hautoberfläche der zu verändernden Körperstelle;
b) Erhalten eines digitalen Modells des Verlaufs der darunter liegenden Knochen;
c) Festlegen des gewünschten Verlaufs der Hautoberfläche der Körperstelle nach der Veränderung;
d) Festlegen der zu verändernden Bereiche der Knochen;
e) Bestimmen von zumindest einer Anpressfläche 2d bezüglich mindestens eines Parameters aus der Gruppe Grösse, Ausrichtung, Oberflächenverlauf;
f) und Herstellen der Formvorrichtung 2 umfassend die Anpressfläche 2d.

Figur 19 zeigt eine sehr leichte, aus Kunststoff gefertigte Formvorrichtung 2 mit entlang der Nasenwurzel umlaufend ausgestaltetem Träger 2a. Die Vorrichtung 1 umfasst zwei Haltevorrichtungen 4,6, einen Gesichtsbogen 5, welcher das erste Haltemittel 4 ausbildet. Das erste Haltemittel 4 ist mit dem Träger 2a fest verbunden und dient im Wesentlichen dazu die Lage der Formvorrichtung 2 bezüglich der Nase möglichst konstant zu halten, indem ein gegenseitiges Verrutschen vermieden wird. Das zweite Haltemittel 6 ist als ein elastisches Band, beispielsweise als Gummiband ausgestaltet, welches über den Befestigungspunkt 6a beim Anpressteil 2c angreift, sodass die einwirkende Kraft im Wesentlichen auf die Anpressfläche 2d einwirkt. Das zweite Haltemittel 6 ist vorzugsweise individuell verstellbar, sodass die auf die Anpressfläche 2d wirkende Kraft über das zweite Haltemittel 6 eingestellt werden kann. Das zweite Haltemittel 6, beispielsweise ausgestaltet als Gummiband, wird auch um den Kopf gelegt, sodass dieses wie das erste Haltemittel 3 auch hinten um den Kopf verläuft.

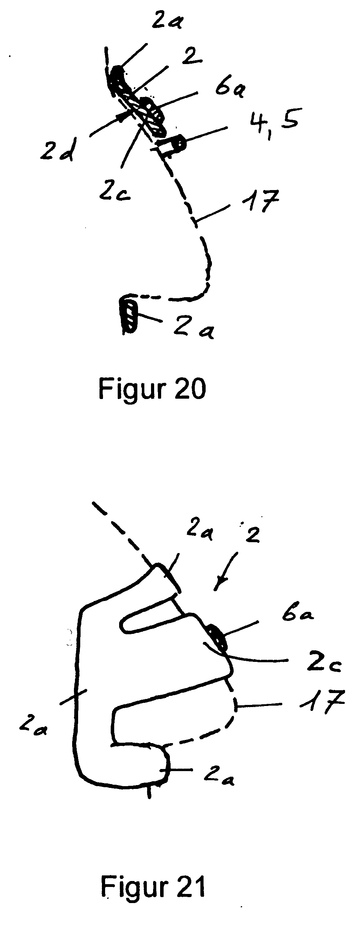

Figur 20 zeigt einen Längsschnitt durch die Figur 19 entlang der Schnittlinie A-A. Die Nase 17 ist schematisch dargestellt. Die Formvorrichtung 2 weist einen Träger 2a auf, welcher umlaufend entlang der Nasenwurzel verläuft, wobei der unten dargestellte Träger 2a zwischen den Nasenflügeln und der Oberlippe verlaufend angeordnet ist. Das Anpressteil 2c ist Teil der einstückigen Formvorrichtung 2 und bildet die Anpressfläche 2d aus, welche im dargestellten Ausführungsbeispiel unmittelbar unter der Befestigungsstelle 6a angeordnet ist.

Figur 21 zeigt in einer Seitenansicht ein weiteres Ausführungsbeispiel einer Formvorrichtung 2 mit Träger 2a, wobei der Träger 2a, wie dargestellt, entlang der Nasenwurzel verläuft, mit Ausnahme des oberen Abschnittes, welcher wie dargestellt über die Nase 17 verläuft. Das Anpressteil 2c ist wieder unmittelbar unterhalt der Befestigung 6a angeordnet. Die in Figur 21 dargestellte Formvorrichtung 2 ist als Spritzgussteil ausgestaltet und weist den Vorteil auf, dass dieses in einer Vielzahl von Ausführungsformen ausgestaltet werden kann. Es können beispielsweise eine Vielzahl von Formvorrichtungen 2 hergestellt werden, welche für unterschiedliche Nasengrössen und/oder Nasenformen geeignet sind. Zudem können beispielsweise eine Vielzahl von Formvorrichtungen 2 hergestellt werden, deren Anpressteile 2c jeweils an unterschiedlichen Orten angeordnet sind.

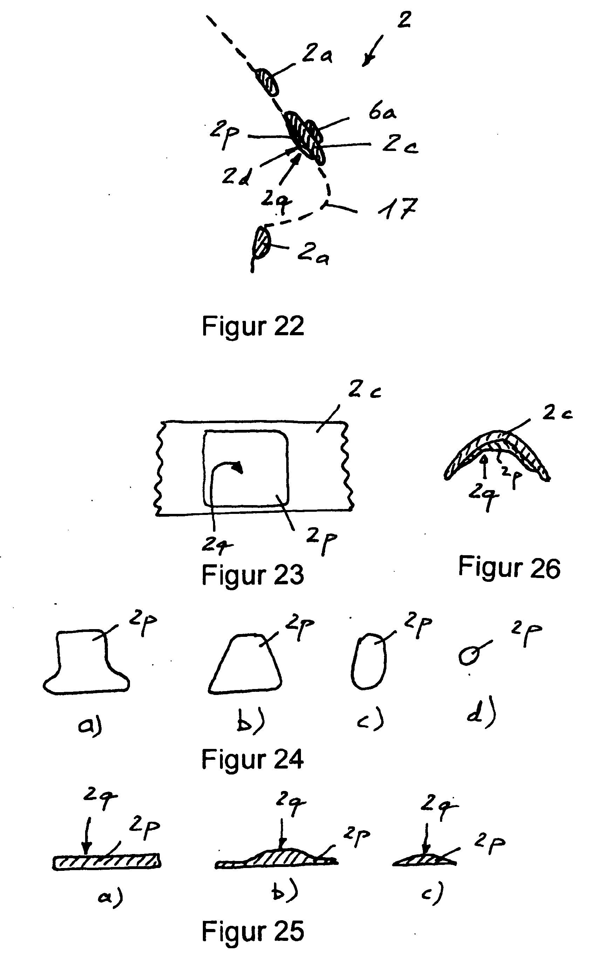

Figur 22 zeigt einen Längsschnitt durch die in Figur 21 dargestellte Formvorrichtung 2. Das Anpressteil 2c muss in der Formvorrichtung 2 jeweils derart angeordnet sein, dass dieses sich an derjenigen Stelle der Nase befinden, an welcher eine Kraft auf die Nase bewirkt werden soll. Daher ist es von Vorteil über eine Vielzahl von Formvorrichtungen 2 zu verfügen, deren Anpressteile 2c unterschiedlich angeordnet sind, sodass das jeweils geeignete Ausgewählt werden kann. Natürlich ist es jedoch auch möglich eine Formvorrichtung 2 individuell auf eine Nase angepasst zu fertigen.

Figur 26 zeigt einen senkrecht zur Betrachtungsebene gemäss Figur 22 verlaufenden Schnitt durch ein Ausführungsbeispiel eines Anpressteils 2c. Der Verlauf der Krümmung, der Öffnungswinkel sowie die Wandstärke des Anpressteils 2c kann in einer Vielzahl von Möglichkeiten variiert werden. Es könnten daher Formvorrichtungen 2 mit einer Vielzahl unterschiedlicher geometrischer Ausgestaltungen zur Verfügung gestellt werden, um beim Anpassen der Formvorrichtung 2 an eine individuelle Nasenform die bezüglich Geometrie am Besten geeignete Formvorrichtung 2 auszuwählen. Die Formvorrichtung 2 könnte jedoch jeweils auch mit einer individuellen Geometrie gefertigt werden, entsprechend der individuellen Nasenform, welche korrigiert werden soll.

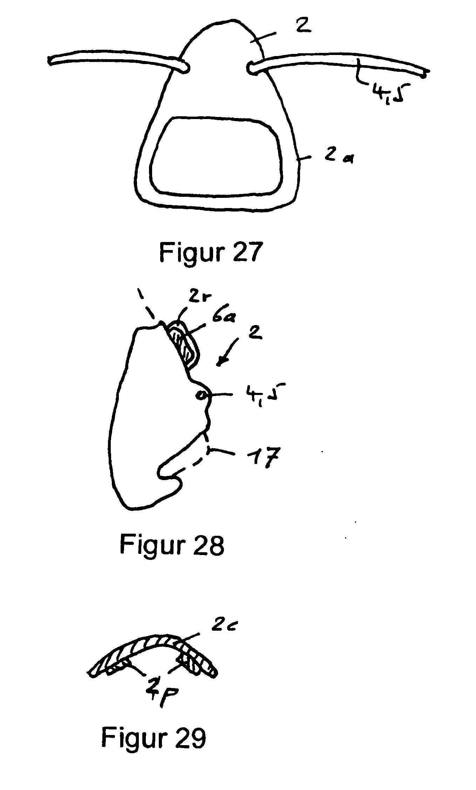

An der Formvorrichtung 2 kann, wie in Figur 22 dargestellt, unten am Anpressteil 2c eine Auflage 2p angeklebt werden, welche die Auflagefläche 2d ausbildet, über welche vorzugsweise die Kraft in die Nase eingeleitet wird. In einer vorteilhaften Ausführungsform wird ein Bausatz zur Verfügung gestellt, umfassend zumindest eine Formvorrichtung 2 sowie eine Mehrzahl von Auflagen 2p, die sich, wie in den Figuren 24a, 24b und 24c dargestellt, bezüglich Geometrie und/oder Fläche als auch, wie in den in Figuren 25a, 25b und 25c dargestellten Schnitten, bezüglich Dickeunterscheiden können. Die Auflage 2p ist auf der einen Seite vorzugsweise mit einem Klebstoff versehen, sodass eine geeignete Auflage 2p ausgewählt und am Anpressteil 2c angeklebt werden kann. Die Grösse, der Ort sowie die Fläche der eingeleiteten Kraft wird nebst dem geometrischen Verlauf des Anpressteils 2c auch durch die entsprechende Wahl der Auflage 2p bestimmt. In einer vorteilhaften Ausgestaltung weist die Auflage 2p eine im Wesentlichen konstante Dicke auf, und ist beispielweise ähnliche wie ein Pflaster ausgestaltet, und besteht beispielsweise aus Kunststoff. Die Auflage 2p könnte jedoch auch wie das in Figur 17 dargestellte Formteil 17k ausgestaltet sein und einerseits eine im Wesentlichen flächige Klebfläche aufweisen, und andererseits eine dreidimensional verlaufende Auflagefläche 2d aufweisen. Vorteilhafterweise stehen zum Anpassen Formvorrichtung 2 an die Nase eines Patienten eine Mehrzahl von Auflagen 2p mit unterschiedlich verlaufenden Auflageflächen 2d und/oder unterschiedlichen Dicken zur Verfügung, sodass die am besten geeignete Auflage 2p in die Formvorrichtung 2 eingeklebt werden kann. Ein derartiger Bausatz weist, wie auch aus dem Schnitt gemäss Figur 26 ersichtlich, den Vorteil auf, dass die Formvorrichtung 2 sehr individuell an eine Nase 17 angepasst werden kann, insbesondere auch der Ort der Krafteinleitung, die Fläche der Krafteinleitung sowie die Stärke der eingeleiteten Kraft. Dies hat zur Folge, dass die erfindungsgemässe Formvorrichtung 2 wenig Schmerzen verursacht, und einen auch langfristig angenehmen Tragkomfort ermöglicht. Dieser langfristig angenehme Tragkomfort ist von zentraler Bedeutung, denn nur ein regelmässiges und länger dauerndes Tragen der Formvorrichtung 2 hat zur Folge, dass sich die Form der Nase bzw. des Knochens verändert. Der erfindungsgemässe Bausatz umfassend zumindest eine individuell ausgesuchte oder individuell angepasste Formvorrichtung 2 sowie eine Mehrzahl geometrisch unterschiedlicher Auflagen 2p weist den wesentlichen Vorteil auf, dass die in der Formvorrichtung 2 befestigte Auflage 2p jederzeit, individuell und schnell ersetzt werden kann, z.B. falls sich eine unangenehme Druckstelle entsteht oder sich ein sonstiges unangenehmes Traggefühl entwickelt. Dadurch ist einerseits sichergestellt, dass der Tragkomfort langfristig gewährleistet ist, und andererseits ist sichergestellt, dass die Druckstelle nicht langfristig übermässig belastet wird, weil die Druckstelle individuell einstellbar und auch individuell veränderbar ist. Diese individuelle Einstellung, die vom jeweiligen Individuum selbst vorgenommen werden kann, ergibt den entscheidenden Vorteil, dass die erfindungsgemässe Formvorrichtung 2 regelmässig, langfristig und mit hohem Tragkomfort getragen wird. Dies ist ein wesentlicher Vorteil, denn nur das regelmässige und langfristige Tragen der Formvorrichtung 2 gewährleistet, dass die Nase sich zur angestrebten Geometrie verformt. Zudem kann eine ständig optimale Krafteinleitung auf die Nase erzielt werden, indem nach einer leichten Verformung der Nase ein andere Auflage 2p verwendet wird, üblicherweise eine dickere Auflage 2p. Dadurch ist gewährleistet dass jeweils die zum Verformen der Nase vorteilhafte oder optimale Kraft an der Nase anliegt, was insbesondere zur Folge haben kann, dass die Formvorrichtung 2 nicht all zu lange getragen werden muss, bis die Nase die angestrebte geometrische Form aufweist. Figur 27 zeigt eine ähnliche, wie in Figur 19 dargestellte Formvorrichtung 2, bei welcher das Verbindungsmittel 4,5 jedoch teilweise innerhalb der Formvorrichtung 2 verläuft.

Figur 28 zeigt, ähnlich wie in Figur 21, eine Seitenansicht eines weiteren Ausführungsbeispiels einer Formvorrichtung 2, wobei das Verbindungsmittel 4,5 wie dargestellt teilweise innerhalb der Formvorrichtung 2 verläuft. Zudem umfasst die Formvorrichtung 2 einen an deren Oberfläche befestigte Öse 2r, durch welche das Haltemittel 6, im dargestellten Ausführungsbeispiel ein Gummiband 6a, verläuft.

Figur 29 zeigt, ähnlich wie Figur 26, einen Schnitt durch die Formvorrichtung 2 mit auf deren Innenseite angeordneten Auflagen 2p. Im Unterschied zum Ausführungsbeispiel gemäss Figur 26 weist das in Figur 29 dargestellte Ausführungsbeispiel zwei separate, beabstandet angeordnete Auflagen 2p auf.

Wirtschaftlich besonders interessant ist der erfindungsgemässe Bausatz, weil üblicherweise eine individuell angepasst Formvorrichtung 2 für die gesamte Behandlung genügt, wogegen eine Mehrzahl geometrisch unterschiedlicher Auflagen 2p erforderlich sind und zur Verfügung gestellt werden, um die Krafteinleitung der sich verformenden Nase oder der jeweiligen geometrischen Form der Formvorrichtung 2 anzupassen. Der erfindungsgemässe Bausatz ist daher in hohem Masse an die individuellen Bedürfnisse anpassbar und trotzdem sehr kostengünstig, da die Auflagen 2p sehr kostengünstig herstellbar sind.

## Patentansprüche

1. Vorrichtung (1) zum Umformen von Nasenknochen, umfassend:
eine Formvorrichtung (2) mit einem Anpressteil (2c), das eine zur Auflage auf die Haut bestimmten Anpressfläche (2d) aufweist,
und eine Haltevorrichtung (3), welche mit der Formvorrichtung (2) verbindbar ist,
wobei die Haltevorrichtung (3) sowie die Formvorrichtung (2) derart zusammenwirkend ausgestaltet ist, dass die Formvorrichtung (2) zumindest an der Anpressfläche (2d) unter Ausübung einer Anpresskraft an der Haut anliegen kann, **dadurch gekennzeichnet, dass** die Formvorrichtung (2) einen Träger (2a) mit einer Auflagefläche (2b) umfasst, und dass der Träger (2a) derart verlaufend ausgestaltet ist,
dass die Auflagefläche (2b) an beiden Seiten der Nase zumindest entlang eines Teilabschnittes der Aussenkontur der Nasenwurzel folgt und über die Auflagefläche (2b) am Gesicht aufliegen kann.

2. Vorrichtung nach Anspruch 1, **dadurch gekennzeichnet, dass** der Träger (2a) derart verlaufend ausgestaltet ist, dass die Auflagefläche (2b) zwischen Oberlippe und Nasenflügeln am Gesicht aufliegen kann, und/oder dass die Auflagefläche (2b) die gesamte Nase umschliesst, und insbesondere entlang der Nasenwurzel verläuft.

3. Vorrichtung nach einem der vorhergehenden Ansprüche,
**dadurch gekennzeichnet, dass** ein Verbindungsmittel (4) am Träger (2a) befestigt ist, und dass das Verbindungsmittel (4) mit der Haltevorrichtung (3) verbindbar ist.

4. Vorrichtung nach einem der vorhergehenden Ansprüche, **dadurch gekennzeichnet, dass** das Anpressteil (2c) mit einer zweiten Haltevorrichtung (6) verbunden ist, und dass die zweite Haltevorrichtung (6) insbesondere als elastisches Band ausgestaltet ist.

5. Vorrichtung nach einem der vorhergehenden Ansprüche, **dadurch gekennzeichnet, dass** das Anpressteil (2c) bezüglich dem Träger (2a) verschiebbar und/oder ersetzbar und/oder fixierbar gelagert ist.

6. Vorrichtung nach einem der vorhergehenden Ansprüche, **dadurch gekennzeichnet, dass** der Träger (2a) zumindest ein Haltemittel (2e) umfasst, und dass das Anpressteil (2c) mit dem Haltemittel (2e) verbunden ist.

7. Vorrichtung nach einem der vorhergehenden Ansprüche, **dadurch gekennzeichnet, dass** das Anpressteil (2c) ein Formteil (2k) umfasst, welches die Anpressfläche (2d) ausbildet.

8. Vorrichtung nach einem der vorhergehenden Ansprüche, **dadurch gekennzeichnet, dass** die Formvorrichtung (2) einstückig ausgebildet ist.

9. Vorrichtung nach einem der vorhergehenden Ansprüche, **dadurch gekennzeichnet, dass** die Anpressfläche (2d) derart in der Formvorrichtung (2) angeordnet und ausgestaltet ist, dass die Anpressfläche (2d) zumindest über einem Teilabschnitt entlang dem Nasensattel anliegt.

10. Verfahren zum Herstellen einer Formvorrichtung (2) für eine Vorrichtung (1) zum Umformen von Knochen nach einem der vorhergehenden Ansprüche, umfassend die Schritte:
a) Erhalten eines digitalen Modells des Verlaufs der Hautoberfläche der zu verändernden Körperstelle;
b) Erhalten eines digitalen Modells des Verlaufs der darunter liegenden Knochen;
c) Festlegen der zu verändernden Bereiche des Knochens;
d) Bestimmen von zumindest einer Anpressfläche (2d) bezüglich mindestens einem Parameter aus der Gruppe Grösse, Ausrichtung, Oberflächenverlauf;
e) und Erstellen der Formvorrichtung (2) umfassend die Anpressfläche (2d).

11. Bausatz zum Erstellen einer Formvorrichtung (2) für eine Vorrichtung (1) zum Umformen von Knochen nach einem der Ansprüche 1 bis 9, wobei die Formvorrichtung (2) einen Träger (2a) mit einer Auflagefläche (2b) umfasst, und wobei der Träger (2a) derart verlaufend ausgestaltet ist, dass die Auflagefläche (2b) an beiden Seiten der Nase zumindest entlang eines Teilabschnittes der Aussenkontur der Nasenwurzel folgt und über die Auflagefläche (2b) am Gesicht aufliegen kann, sowie umfassend eine Mehrzahl unterschiedlich ausgestalteter Anpressteile (2c), welche mit dem Träger (2a) verbindbar sind, wobei jedes Anpressteil (2c) eine zur Auflage auf die Haut bestimmte Anpressfläche (2d) aufweist.

12. Bausatz zum Erstellen einer Formvorrichtung (2) für eine Vorrichtung (1) zum Umformen von Knochen nach einem der Ansprüche 1 bis 9, wobei die Formvorrichtung (2) einstückig ausgestaltet ist und einen Träger (2a) mit einer Auflagefläche (2b) sowie ein Anpressteil (2c) umfasst, und wobei der Träger (2a) derart verlaufend ausgestaltet ist, dass die Auflagefläche (2b) an beiden Seiten der Nase zumindest entlang eines Teilabschnittes der Aussenkontur der Nasenwurzel folgt und über die Auflagefläche (2b) am Gesicht aufliegen kann, sowie umfassend zumindest eine Auflage (2p) mit einer Auflagefläche (2q), wobei die Auflage (2p) derart mit dem Anpressteil (2c) verbindbar ist, dass die Auflagefläche (2q) eine zur Auflage auf die Haut bestimmte Anpressfläche (2d) ausbildet.

13. Bausatz nach Anspruch 12, **dadurch gekennzeichnet, dass** die Auflage (2p) Pflaster ähnlich ausgebildet ist, indem die eine Seite der Auflage (2p) die Auflagefläche (2q) ausbildet und die andere Seite mit Klebstoff versehen ist, welcher zur Befestigung am Anpressteil (2c) der Formvorrichtung (2) dient.

14. Bausatz nach Anspruch 12 oder 13 umfassend eine Mehrzahl geometrisch unterschiedlich ausgestalteter Auflagen (2p).

15. Bausatz nach Anspruch 14, **dadurch gekennzeichnet, dass** die Auflagen (2p) eine unterschiedliche Dicke aufweisen und/oder dass deren Auflageflächen (2q) eine unterschiedliche Geometrie und/oder eine unterschiedliche Gesamtfläche aufweisen.

## Claims

1. A device (1) for shaping nasal bones, comprising:
a shaping device (2) with a pressing part (2c) having a pressing surface (2d) for placement on the skin,
and a holding device (3) which is connectable to the shaping device (2),
wherein each of the holding device (3) and the shaping device (2) is formed so as to provide a cooperative capability of causing at least the pressing surface (2d) of the shaping device (2) to rest against the skin exerting a pressing force, **characterised in that** the shaping device (2) comprises a carrier (2a) with a contact surface (2b), and **in that** the carrier (2a) has a form that allows resting on the face via the contact surface (2b) and extends such that the contact surface (2b) follows at least a sub-section of the outer contour of the root of the nose on both sides of the nose.

2. A device in accordance with claim 1, **characterised in that** the carrier (2a) is configured to extend so as to allow the contact surface (2b) to rest on the face between the upper lip and the alae of the nose and/or such that the contact surface (2b) surrounds the whole nose and, in particular, extends along the root of the nose.

3. A device in accordance with one of the preceding claims, **characterised in that** a connection means (4) is fastened to the carrier (2a), and **in that** the connection means (4) can be connected to the holding device (3).

4. A device in accordance with one of the preceding claims, **characterised in that** the pressing part (2c) is connected with a second holding device (6), and **in that** the second holding device (6), in particular, takes the form of an elastic band.

5. A device in accordance with one of the preceding claims, **characterised in that**, relative to the carrier (2a), the pressing part (2c) is displaceable and/or replaceable and/or fixably mounted.

6. A device in accordance with one of the preceding claims, **characterised in that** the carrier (2a) comprises at least one holding means (2e), and **in that** the pressing part (2c) is connected with the holding means (2e).

7. A device in accordance with one of the preceding claims, **characterised in that** the pressing part (2c) comprises a shaping part (2k) which forms the pressing surface (2d).

8. A device in accordance with one of the preceding claims, **characterised in that** the shaping device (2) is a single-piece construction.

9. A device in accordance with one of the preceding claims, **characterised in that** the pressing surface (2d) is arranged and configured in the shaping device (2) such that the pressing surface (2d) rests along at least a sub-section of the bridge of the nose.

10. A method of manufacturing a shaping device (2) for a device (1) for shaping bones according to one of the preceding claims comprising the steps:
a) acquisition of a digital model of the layout of the skin surface at the location on the body which is to be modified;
b) acquisition of a digital model of the layout of the underlying bones;
c) definition of the regions of the bone to be modified;
d) specification of at least one pressing surface (2d) in terms of at least one parameter out of the set of size, orientation, surface layout;
e) and fabrication of the shaping device (2) comprising the pressing surface (2d).

11. A kit for the fabrication of a shaping device (2) for a device (1) for shaping bones according to one of claims 1 to 9, wherein the shaping device (2) comprises a carrier (2a) having a contact surface (2b), and wherein the carrier (2a) has a form that allows resting on the face via the contact surface (2b) and extends such that the contact surface (2b) follows at least a sub-section of the outer contour of the root of the nose on both sides of the nose and comprising a plurality of differently configured pressing parts (2c) which are connectable to the carrier (2a), wherein each pressing part (2c) has a pressing surface (2d) for placement on the skin.

12. A kit for the fabrication of a shaping device (2) for a device (1) for shaping bones according to one of claims 1 to 9, wherein the shaping device (2) is a single-piece construction and comprises a carrier (2a) with a contact surface (2b) and a pressing part (2c), and wherein the carrier (2a) has a form that allows resting on the face via the contact surface (2b) and extends such that the contact surface (2b) follows at least a sub-section of the outer contour of the root of the nose on both sides of the nose, and comprising at least one overlay (2p) having an overlay surface (2q), wherein the overlay (2p) is connectable with the pressing part (2c) such that the overlay surface (2q) forms a pressing surface (2d) for placement on the skin.

13. A kit in accordance with claim 12, **characterised in that** the overlay (2p) takes a form similar to a plaster **in that** one side of the overlay (2p) forms the overlay surface (2q) and the other side is provided with adhesive which serves for fixation on the pressing part (2c) of the shaping device (2).

14. A kit in accordance with claim 12 or 13 comprising a plurality of geometrically differently configured overlays (2p).

15. A kit in accordance with claim 14, **characterised in that** the overlays (2p) have different thicknesses, and/or **in that** their overlay surfaces (2q) have different geometries and/or different overall areas.

## Revendications

1. Dispositif (1) de remodelage des os nasaux, comprenant :
un dispositif de remodelage (2) muni d'un élément de pression (2c) qui comprend une surface de pression (2d) conçue pour être appliquée sur la peau ;
et un dispositif de maintien (3) qui peut être raccordé au dispositif de remodelage (2) ;
dans lequel le dispositif de maintien (3), de même que le dispositif de remodelage (2), sont configurés pour coopérer de telle sorte que le dispositif de remodelage (2) peut entrer en contact avec la peau au moins au niveau de la surface de pression (2d) tout en exerçant une force de pression, **caractérisé en ce que** le dispositif de remodelage (2) comprend un support (2a) possédant une surface d'appui (2b), et **en ce que** le support (2a) est formé pour s'étendre de telle sorte que la surface d'appui (2b) suit de part et d'autre du nez le contour externe de la racine du nez au moins le long d'une section partielle et peut reposer sur le visage au moyen de la surface d'appui (2b).

2. Dispositif selon la revendication 1, **caractérisé en ce que** le support (2a) est configuré pour s'étendre de telle sorte que la surface d'appui (2b) peut reposer sur le visage entre la lèvre supérieure et les ailes du nez, et/ou que la surface d'appui (2b) entoure le nez entier et, en particulier, s'étend le long de la racine du nez.

3. Dispositif selon l'une quelconque des revendications précédentes, **caractérisé en ce qu'**un moyen de raccord (4) est fixé au support (2a), et **en ce que** le moyen de raccord (4) peut être relié au dispositif de maintien (3).

4. Dispositif selon l'une quelconque des revendications précédentes, **caractérisé en ce que** l'élément de pression (2c) est raccordé à un second dispositif de maintien (6), et **en ce que** le second dispositif de maintien (6) est particulièrement réalisé sous la forme d'une bande élastique.

5. Dispositif selon l'une quelconque des revendications précédentes, **caractérisé en ce que** l'élément de pression (2c) est monté avec faculté de déplacement et/ou de remplacement et/ou de fixation par rapport au support (2a).

6. Dispositif selon l'une quelconque des revendications précédentes, **caractérisé en ce que** le support (2a) comprend au moins un moyen de maintien (2e), et **en ce que** l'élément de pression (2c) est raccordé au moyen de maintien (2e).

7. Dispositif selon l'une quelconque des revendications précédentes, **caractérisé en ce que** l'élément de pression (2c) comprend un élément de remodelage (2k) qui forme la surface de pression (2d).

8. Dispositif selon l'une quelconque des revendications précédentes, **caractérisé en ce que** le dispositif de remodelage (2) est formé en élément monobloc.

9. Dispositif selon l'une quelconque des revendications précédentes, **caractérisé en ce que** la surface de pression (2d) est agencée et configurée dans le dispositif de remodelage (2) de telle sorte que la surface de pression (2d) entre en contact au moins sur une section partielle le long du bloc nasal.

10. Procédé de fabrication d'un dispositif de remodelage (2) pour un dispositif (1) de remodelage d'os selon l'une quelconque des revendications précédentes, comprenant les étapes consistant à:
a) obtenir un modèle numérique de l'étendue de la surface de peau du point corporel à modifier ;
b) obtenir un modèle numérique de l'étendue de l'os situé en dessous ;
c) spécifier les régions de l'os à modifier ;
d) déterminer au moins une surface de pression (2d) sous le rapport d'au moins un paramètre du groupe taille, alignement, étendue surfacique ;
e) et préparer le dispositif de remodelage (2) comprenant la surface de pression (2d).

11. Kit pour la préparation d'un dispositif de remodelage (2) pour un dispositif (1) de remodelage d'os selon l'une quelconque des revendications 1 à 9, dans lequel le dispositif de remodelage (2) comprend un support (2a) possédant une surface d'appui (2b), et dans lequel le support (2a) est formé pour s'étendre de telle sorte que la surface d'appui (2b) suit de part et d'autre du nez le contour externe de la racine du nez au moins le long d'une section partielle et peut reposer sur le visage au moyen de la surface d'appui (2b), et comprenant une pluralité d'éléments de pression (2c) différemment configurés qui peuvent être raccordés au support (2a), chaque élément de pression (2c) comprenant une surface de pression (2d) conçue pour être appliquée sur la peau.

12. Kit pour la préparation d'un dispositif de remodelage (2) pour un dispositif (1) de remodelage d'os selon l'une quelconque des revendications 1 à 9, dans lequel le dispositif de remodelage (2) est formé en élément monobloc et comprend un support (2a) doté d'une surface d'appui (2b) ainsi qu'un élément de pression (2c), et dans lequel le support (2a) est formé pour s'étendre de telle sorte que la surface d'appui (2b) suit de part et d'autre du nez le contour externe de la racine du nez au moins le long d'une section partielle et peut reposer sur le visage au moyen de la surface d'appui (2b), et comprenant au moins un élément de recouvrement (2p) possédant une surface d'appui (2q), l'élément de recouvrement (2p) pouvant être raccordé à l'élément de pression (2c) de telle sorte que la surface d'appui (2q) forme une surface de pression (2d) conçue pour être appliquée sur la peau.

13. Kit selon la revendication 12, **caractérisé en ce que** l'élément de recouvrement (2p) est réalisé de manière similaire à un patch, par le fait qu'une face de l'élément de recouvrement (2p) forme la surface d'appui (2q) et que l'autre face est munie d'un adhésif qui sert à la fixation à l'élément de pression (2c) du dispositif de remodelage (2).

14. Kit selon la revendication 12 ou 13 comprenant une pluralité d'éléments de recouvrement (2p) présentant des configurations géométriquement différentes.

15. Kit selon la revendication 14, **caractérisé en ce que** les éléments de recouvrement (2p) possèdent des épaisseurs différentes, et/ou **en ce que** leurs surfaces d'appui (2q) présentent des géométries différentes et/ou des aires surfaciques totales différentes.
